(19) Europäisches Patentamt / European Patent Office / Office européen des brevets

(11) **EP 3 303 524 B1**

(12) **EUROPEAN PATENT SPECIFICATION**

(45) Date of publication and mention of the grant of the patent:
**27.04.2022 Bulletin 2022/17**

(21) Application number: **16727818.3**

(22) Date of filing: **03.06.2016**

(51) International Patent Classification (IPC):
*C10L 3/08* (2006.01)      *C25B 1/04* (2021.01)

(52) Cooperative Patent Classification (CPC):
**C10L 3/08; C25B 1/04;** C10L 2200/0469;
C10L 2290/02; C10L 2290/04; C10L 2290/06;
C10L 2290/36; C10L 2290/38; Y02E 60/36;
Y02P 20/582

(86) International application number:
**PCT/GB2016/051647**

(87) International publication number:
**WO 2016/193751 (08.12.2016 Gazette 2016/49)**

(54) **PROCESS FOR PRODUCING A SUBSTITUTE NATURAL GAS FROM SYNTHESIS GAS**

VERFAHREN ZUR HERSTELLUNG EINES ERDGASSUBSTITUTS AUS SYNTHESEGAS

PROCÉDÉ DE PRODUCTION D'UN GAZ NATUREL DE SUBSTITUTION À PARTIR D'UN GAZ DE SYNTHÈSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2015 GB 201509684**

(43) Date of publication of application:
**11.04.2018 Bulletin 2018/15**

(73) Proprietor: **Advanced Plasma Power Limited**
**Swindon, Wiltshire SN3 4DE (GB)**

(72) Inventors:
• **CHAPMAN, Chris**
**Swindon, Wiltshire SN3 4DE (GB)**

• **MATERAZZI, Massimiliano**
**Swindon, Wiltshire SN3 4DE (GB)**

(74) Representative: **Boult Wade Tennant LLP**
**Salisbury Square House**
**8 Salisbury Square**
**London EC4Y 8AP (GB)**

(56) References cited:
WO-A1-2012/067754      WO-A1-2015/011503
WO-A2-2008/002593      DE-A1-102012 010 542
DE-A1-102012 015 788      DE-A1-102012 200 221
US-A1- 2007 178 034

**Description**

The invention relates to a process for producing a substitute natural gas.

Substitute natural gas (SNG) can be produced from fossil fuels such as coal, and it is known to incorporate SNG together with natural gas in a gas grid. Substitute natural gas obtained from biogenic feedstocks is also known and is termed bio-SNG. In view of the need to employ more renewable sources of energy, it is proposed to distribute SNG and bio-SNG together with natural gas in a gas grid.

It is known to manufacture SNG from a synthesis gas (a gas mixture comprising primarily hydrogen and carbon monoxide). Synthesis gases may be obtained, for example, by the gasification of a hydrocarbon feedstock. In a typical SNG manufacturing method, the synthesis gas is subjected to a methanation reaction to convert carbon monoxide into methane.

Typical synthesis gases have a hydrogen to carbon monoxide ratio in the range of 0.7-1.2:1. Since methanation requires a hydrogen to carbon monoxide ratio of 3:1, the ratio of hydrogen to carbon monoxide in the synthesis gas is typically increased prior to methanation by reacting a portion of the carbon monoxide with water to produce hydrogen and carbon dioxide. This is known as the "water-gas-shift" reaction. While this increase in the hydrogen : carbon monoxide ratio increases the amount of methane formed during the subsequent methanation, it results in around two thirds of the carbon content of the synthesis gas being jettisoned from the process as carbon dioxide. This results in the process being costly and chemically wasteful, and having high carbon emissions. Furthermore, the process requires a number of purification steps to separate the methane from the residual carbon dioxide, thereby adding to the complexity of the process.

WO2015/120983A1 relates to a method and system of producing biomethane. CN204342750U relates to a structure for preparing natural gas through utilisation of electrolysis hydrogen preparation and synthesis gas from coal. WO00/21911A1 relates to a process for converting hydrogen into substitute natural gas. GB2485923A relates to a process for increasing the hydrogen content in a synthesis gas. EP2843031A1 relates to gas-steam efficient cogeneration process and system based on biomass gasification and methanation. EP2236457A1 relates to a process for producing a purified synthesis gas. US2010/0286292A1 relates to a process for the production of substitute natural gas. US2010/0272619A1 relates to a method and apparatus for substitute natural gas generation.

DE102012200221 discloses the production of a methane-rich gas, useful as fuel for fuel cell. DE102012015788 discloses energy generation and storage by methane combustion and synthesis.

WO2012/067754 discloses a process for making a synthetic natural gas. DE102012010542 discloses a process for the production of a synthesis gas. WO2015/011503 discloses a process for making a synthetic natural gas.

The present invention seeks to tackle at least some of the constraints associated with the prior art or at least to provide a commercially acceptable alternative solution thereto.

In a first aspect, the present invention provides a process for producing a substitute natural gas, the process comprising the steps of:

(1) providing a synthesis gas comprising hydrogen and carbon monoxide;
(2) forming a hydrogen-enriched synthesis gas;
(3) subjecting the hydrogen-enriched synthesis gas to a methanation reaction to convert at least a portion of the gas into methane thereby forming a methane-enriched gas; and
(4) recovering from the methane-enriched gas a methane-containing gas, wherein step (2) comprises providing a hydrogen gas and combining the hydrogen gas with the synthesis gas, wherein:

(i) at least some of the hydrogen gas is produced by the decomposition of ammonia and/or
(ii) at least some of the hydrogen gas is produced by steam reforming of a hydrocarbon feedstock.

The process of the present invention may exhibit improved conversion rates and may require fewer refining and/or purification steps. Furthermore, the process may enable control of the highly exothermic methanation reaction, leading to improved safety whilst increasing overall process performance. In addition, the process is highly sustainable and typically generates no carbon dioxide emissions. The invention may also provide an effective electricity grid balancing function.

Each aspect or embodiment as defined herein may be combined with any other aspect(s) or embodiment(s) unless clearly indicated to the contrary. In particular, any features indicated as being preferred or advantageous may be combined with any other feature indicated as being preferred or advantageous.

The term "substitute natural gas" or "SNG" as used herein encompasses a gas comprising primarily methane.

The term "synthesis gas" or "syngas" as used herein encompasses a gas mixture comprising primarily hydrogen and carbon monoxide. It may also comprise gaseous species such as carbon dioxide, water vapour and nitrogen, which would together typically not exceed 30 vol.%. It may also contain impurities such as, for example, solid particulate and tarry species. The amount of these impurities present will typically not exceed 5 %w/w.

The term "water-gas-shift reaction" as used herein encompasses a reaction in which carbon monoxide reacts with water vapour to form carbon dioxide and hydrogen, i.e.

$$CO_{(g)} + H_2O_{(v)} \rightarrow CO_{2(g)} + H_{2(g)}$$

The term "methanation reaction" as used herein encompasses a reaction in which carbon monoxide reacts with hydrogen to form methane and water, i.e.

$$CO_{(g)} + 3H_{2(g)} \rightarrow CH_{4(g)} + H_2O_{(g)}$$

The term "Sabatier reaction" as used herein encompasses a reaction in which carbon dioxide reacts with hydrogen to form methane and water, i.e.

$$CO_{2(g)} + 4H_{2(g)} \rightarrow CH_{4(g)} + 2H_2O_{(g)}$$

The term "steam reforming" as used herein encompasses a reaction in which an alkane, such as methane, is reacted with water to produce hydrogen and carbon monoxide, i.e.

$$CH_{4(g)} + H_2O_{(g)} \rightarrow CO_{(g)} + 3H_{2(g)}$$

The term "Wobbe number" as used herein is defined as:

$$I_W = \frac{V_C}{\sqrt{G_S}}$$

where $I_W$ is the Wobbe number, $V_C$ is the higher heating value or higher calorific value, and $G_S$ is the specific gravity. The Wobbe number may be calculated by the appropriate methodology such as ISO 6976. The Wobbe number (sometimes referred to as Wobbe index) provides an indication of the interchangeability of fuel gases and is universally used as a determinant in gas quality specifications used in gas network or transportation utilities. In physical terms the Wobbe number compares the combustion energy output of fuel gases of varying composition for an appliance (i.e. boiler or cooker) whereby two fuels having an identical Wobbe number will also have the same energy output (assuming all other factors such as pressure and flow rate are kept constant). The Wobbe number is especially important when considering the impact of injecting SNG into the gas grid.

Prior to step (2) and/or step (3), the synthesis gas may be cleaned in a series of stages to remove various contaminant species that would otherwise poison the downstream catalytic processes. The contaminants that may need to be removed will vary depending on the chemical composition of the feedstock and the conditions under which it is converted into synthesis gas. Typical contaminants include sulphur and chloride species, tars, unsaturated hydrocarbons, heavy metals and particulates. Such contaminant species may be removed, for example, by physical or chemical absorption/adsorption. Prior to step (3), the synthesis gas may undergo "polishing". For example, a guard bed (e.g. a ZnO guard bed), may be used to reduce sulphur and chlorine impurities to below 20 ppb, thereby minimising the rate of poisoning of catalysts used in the methanation reaction.

As discussed above, the methanation reaction tends to completion with a hydrogen to carbon monoxide ratio in excess of 3:1. Typical synthesis gases have a hydrogen ($H_2$) to carbon monoxide ratio in the range of 0.7-1.2:1. The enrichment of the synthesis gas in step (2) increases the ratio of hydrogen to carbon, thereby increasing the amount of methane that can be generated in the methanation reaction of step (3). Accordingly, the conversion rate of the process is increased.

Step (2) comprises providing a hydrogen gas and combining the hydrogen gas with the synthesis gas (i.e. adding additional hydrogen gas to the synthesis gas). In other words, in contrast to prior art SNG production methods, the additional hydrogen gas is not generated *in situ,* i.e. it is generated externally. In addition, the additional hydrogen gas is not generated by reaction of species contained in the synthesis gas, such as carbon monoxide. This enables the amount of hydrogen to be controlled, as well as the ratios of hydrogen to carbon monoxide and carbon dioxide. In conventional SNG production processes, hydrogen is generated *in situ* via a water-gas-shift reaction. While the water-gas-shift reaction increases the ratio of hydrogen to carbon monoxide, it does so by generating carbon dioxide as a by-product. The amount of hydrogen generated by the water-gas-shift reaction can never be enough to drive the process to completion (i.e. the complete conversion of all carbon monoxide and carbon dioxide to methane). This results in around two thirds of the carbon contained in the synthesis gas being jettisoned, thereby decreasing the carbon conversion rate, and results in high levels of carbon emissions.

In contrast to conventional SNG production processes, the process of the present invention may provide an excess of hydrogen in the methanation reaction, thereby allowing near complete conversion of the carbon monoxide into methane. Accordingly, in comparison to conventional SNG production processes, the process of the present invention produces 2 to 3 times more methane from the same synthesis gas. Furthermore, in view of the excess hydrogen present, any carbon dioxide contained in the synthesis gas may also be converted to methane, for example via the Sabatier reaction. Accordingly, in contrast to conventional SNG production processes, the process of the present invention may result in complete conversion of the carbon content of the synthesis gas into methane. Accordingly, the process of the present invention is highly efficient, and also typically generates no carbon dioxide emissions. In addition, since the methane-enriched gas typically comprises substantially only methane and water, few, if any, subsequent processing and/or purification steps are required. Accordingly, in contrast to conventional SNG production processes, the process of the present invention is simplified.

As discussed above, the hydrogen content of the hydrogen-enriched synthesis gas may be in excess of the carbon-content (i.e. the carbon monoxide and carbon dioxide). The levels of carbon monoxide and carbon dioxide will vary depending on the source and/or production method of the synthesis gas. In view of the differing hydrogen : carbon species ratios required by the methanation reaction and Sabatier reaction, an "excess" of hydrogen in the hydrogen-enriched gas will be obtained when the following equation is satisfied:

$$[H_2] > 3[CO] + 4[CO_2]$$

The methanation reaction is preferably conducted at a temperature of from 200 to 450 °C. Such temperatures allow flexibility of reactor design which can therefore be operated under isothermal or adiabatic conditions (or a combination of reactors operating in series).

The methanation reaction may be carried out in the presence of, for example, a transition metal catalyst such as, for example, a nickel-containing catalyst or an iron-containing catalyst. An example of a suitable catalyst for the methanation catalyst is a Johnson Matthey commercial methanation catalyst in pellet form - Katalco 11-4m containing 22% Ni (metallic basis). The catalysts may be supported on, for example, alumina, silica or zeolite substrates.

In step (3), at least a portion of the hydrogen-enriched gas is converted into methane. Typically the majority of the carbon monoxide in the hydrogen-enriched synthesis gas is converted to methane, more typically substantially all of the carbon monoxide in the hydrogen-enriched synthesis gas is converted to methane.

In step (3), in order to ensure high conversion efficiencies to methane, a multistage reactor may be used, for example employing a nickel catalyst. The reactor temperature may be controlled by a number of techniques including: multi stage injection of the reactant, recycling a part of the product stream to control the extent of the equilibrium reaction and the use of inter or intra stage cooling. The reactor design configurations and catalyst materials employed may be altered depending on the specific process chemistry and thermal rating of the facility. The methanation reaction is highly exothermic, and is challenging to control in a conventional process. The process of the present invention may include independent addition of hydrogen at multiple stages. For the preliminary stages this may be sub-stoichiometric, thereby enabling control of both the extent of the exotherm and the maximum temperature attained within the reactor. This control is not possible in a conventional process employing a water-gas-shift reaction, since the hydrogen is generated within the synthesis gas.

The methanation reaction forms methane and water. The methane-containing gas of step (4) may be recovered from the methane-enriched gas by condensation of the water from the methane-enriched gas. This may occur, for example, by reducing the temperature and/or increasing the pressure of the methane-enriched gas.

The methane-containing gas recovered in step (4) typically comprises predominantly methane, more typically at least 80 vol.% methane, even more typically at least 90 vol. % methane, still even more typically at least 95 vol.% methane. The methane-enriched gas may also comprise gases other than methane, for example small amounts of nitrogen and small amount of hydrogen (for example, unreacted hydrogen from the methanation reaction). In one example, the methane enriched gas comprises about 96 vol.% methane, the remainder being nitrogen, hydrogen and any unavoidable impurities.

The methane-containing gas recovered in step (4) may be used as a -substitute natural gas. The methane-containing gas may be subjected to further processing before being used as a substitute natural gas, for example pressurisation and/or purification.

The methane-containing gas recovered in step (4) exhibits similar properties to that of natural gas, and is therefore suitable to be combined with natural gas in a gas grid. It may also be suitable for use as a transport fuel, for example as a substitute compressed natural gas (CNG) or liquefied natural gas (LNG). The methane-containing gas recovered in step (4) preferably exhibits a gross calorific value (GCV) of from 35 to 45 MJ/m$^3$, more preferably from 36.9 to 42.3 MJ/m$^3$. Such GCV values are similar to natural gas. The methane-containing gas preferably has a Wobbe number of from 45 to 55 MJ/M$^3$, more preferably from 47.2 to 51.4 MJ/m$^3$. This makes the methane-containing gas more suitable for incorporation into a gas grid.

The hydrogen gas in step (2) may be conveyed to and/or stored on site. Hydrogen is a light gas and a low density energy source. Hydrogen is currently difficult and costly to contain and consequently to transport. Accordingly, the hydrogen gas is preferably generated on-site and preferably as required. The hydrogen may be generated, for example, from the electrolysis of water, the degradation of ammonia and/or steam reforming of a hydrocarbon feedstock. Such processes are discussed in more detail below. At least some of the hydrogen gas is produced by the decomposition of ammonia and/or by steam reforming of a hydrocarbon feedstock. Water, ammonia and hydrocarbon feedstocks are easier to convey and to store than hydrogen. The hydrogen gas may be generated in a continuous process. Alternatively, the hydrogen gas may be generated in a batch process. The use of a batch process allows the hydrogen to be generated using, for example, low cost energy (e.g. during periods of oversupply) and/or renewable energy that is prone to periods of oversupply (e.g. wind, solar, tidal, hydroelectric etc.). The ammonia and hydrocarbon feedstocks may be re-generated. Hydrogen gas required for such regeneration may be obtained from the electrolysis of water. The hydrogen gas production required for the subsequent regeneration of the ammonia or hydrocarbon feedstock as well as the regeneration itself may be carried out in a batch process. Accordingly, this process may advantageously make use of cheap energy and/or renewable energy prone to periods of oversupply as well as avoid the requirement to store hydrogen.

The process is preferably a continuous process. The synthesis gas is preferably generated from biomass. Preferably, the hydrogen gas is generated from non-biogenic sources.

In a preferred embodiment, at least some of the hydrogen gas is produced via the electrolysis of water. Hydrogen may be generated on demand by the electrolysis of water. This is advantageous since water is much easier to store than hydrogen gas.

In one embodiment according to the invention, at least some of the hydrogen gas is produced by the decomposition of ammonia. Hydrogen may be generated on demand by the decomposition of ammonia. This is advantageous since ammonia (typically liquid ammonia) is much easier to store than hydrogen gas. Hydrogen production via the decomposition of ammonia may occur in a number of ways including, for example, thermal decomposition and/or catalytic cracking. Catalysts suitable for use in the catalytic cracking include, for example, those involved in fuel cells, for example, Ni/Al$_2$O$_3$, NiCeO$_2$/Al$_2$O$_3$, Cr$_2$O$_3$, Ru/ZrO$_2$, and Ru on carbon nano-fibres. For these catalysts, a minimum temperature of 300 °C is typically required for efficient release of ammonia for hydrogen production. Electrolysis or electro-oxidation is another method of extracting hydrogen from ammonia. It has the advantage of scalability and versatility to interface with renewable energy sources including those whose electricity production varies with time. The theoretical thermodynamic energy consumption is 1.55 Wh/g of H$_2$ from electrolysis of NH$_3$ compared to 33 Wh/g of H$_2$ from H$_2$O. This means that, theoretically, ammonia electrolysis consumes 95% less energy to produce a quantity of hydrogen than water electrolysis. However, this does not account for kinetics of the reaction. Preferably, the majority of the hydrogen gas is produced via the decomposition of ammonia, more preferably substantially all of the hydrogen gas is produced by the decomposition of ammonia.

The ammonia is preferably generated and/or regenerated using the Haber-Bosch process and/or solid state ammonia synthesis. The Haber-Bosch process is well known in the art, and typically involves combining hydrogen and nitrogen gases over iron-based catalysts at about 500 °C and under 200 to 300 atmospheres of pressure to produce ammonia. Typically, only a fraction of the gas is converted to ammonia in a single pass through the converter due to thermodynamic equilibrium of the reaction. The remaining unreacted gas is typically passed through the converter again, forming a flow loop for the unreacted gas. In a typical set-up, refrigeration coolers decrease the temperature of the gas to -10 °C to -25 °C so that the ammonia condenses out of the mixture, thus leaving behind the unreacted gas. In this approach to ammonia production no CO$_2$ is produced, provided the electricity to power the electrolysis, and later the Haber-Bosch reaction, is derived from a non-polluting source of electricity (such as hydropower). Solid state ammonia synthesis, or "SSAS", uses a solid state electrochemical process to produce ammonia from nitrogen, water, and electricity (see, for example, Ganley J, Holbrook J & McKinley D. Solid state ammonia synthesis. Oct, 2007. Presented at the Ammonia - Sustainable Emission-Free Fuel Conference, San Francisco, CA. October 15-16, 2007, the disclosure of which is hereby incorporated by reference). The process involves water being broken into oxygen and hydrogen with the hydrogen reacting with nitrogen to form ammonia. The SSAS technology is ideally suited for renewable energy sources that produce electricity, such as wind and solar photovoltaic, since electrolysers for hydrogen production and the Haber-Bosch synloop are eliminated with the SSAS system, resulting in several energy and economic benefits.

Preferably, the ammonia is generated using the Haber-Bosch process, and the hydrogen for use in the Haber-Bosch process is generated via the electrolysis of water. Hydrogen may be generated on-demand via the electrolysis of water, which is easier to store than hydrogen. The hydrogen may be generated when the cost of electricity is cheap (e.g. during periods of oversupply). In addition, the hydrogen may be generated using power from renewable sources such as, for example, wind, solar, tidal and hydroelectric dams. Such renewable sources provide varied outputs depending on the prevailing conditions, and thereby are prone to periods of oversupply. However, since the ammonia is typically easy to store, the generation of hydrogen and subsequent conversion to ammonia may be carried out solely during such periods of oversupply. This may reduce the cost and/or carbon footprint of the process. In other words, while the SNG production may be a continuous process, the hydrogen generation and subsequent conversion to ammonia may be a batch process restricted to periods in which electricity is cheap and/or from a renewable source. This is a particularly significant advantage of the present invention.

Oxygen generated by the electrolysis of water may be reacted with a feedstock to produce synthesis gas for step (1). Typical synthesis gas production methods, such as, for example, gasification and/or gas-plasma methods, often require a source of highly pure oxygen. Oxygen generated by electrolysis is highly pure, thereby avoiding the need to incur the cost of procuring a source of highly pure oxygen.

The nitrogen for use in the Haber-Bosch process may be obtained using an air separation unit. Oxygen separated in the air separation unit may also be reacted with a fuel to produce synthesis gas for step (1).

At least some of the hydrogen gas may be obtained from an orphan hydrogen rich stream. This may provide a useful clean-up mechanism for such streams. At least some of the hydrogen gas may be obtained from the pyrolysis of, for example, coke. At least some of the hydrogen gas may be obtained from pyrolysis, for example, in a carbon black manufacturing method. Preferably at least some of the hydrogen gas is obtained from pyrolysis or cracking of hydrocarbons, more preferably wherein the pyrolysis and/or cracking of hydrocarbons is part of a carbon black manufacturing method.

In an alternative embodiment according to the invention, at least some of the hydrogen gas is produced by steam reforming of a hydrocarbon feedstock. Hydrocarbon feedstocks for use in the present invention may be solid (e.g. coal, coke, biomass), liquid (e.g. higher alkanes, naphthas) or gaseous (e.g. lower alkanes). Hydrogen may be generated on demand from a hydrocarbon feedstock, which is easier to store than hydrogen. The hydrocarbon feedstock is preferably methane. Methane is cheap, readily available and easy to store. In addition, methane is already part of the existing infrastructure: bringing methane to the plant and taking SNG away from it. Preferably, the majority of the hydrogen gas is produced by steam reforming of a hydrocarbon feedstock, more preferably substantially all of the hydrogen gas is produced by steam reforming of a hydrocarbon feedstock.

Steam reforming, such as steam methane reforming (SMR), is highly endothermic, and is typically carried out at temperatures of from 700 to 1000 °C using suitable catalysts known in the art (e.g. nickel-based catalysts). The reaction may be carried out allothermally (energy supplied by an external source) or autothermally (energy produced internally by combustion of a methane fraction). In the latter case, a supply of pure oxygen is also required to partially combust the gas and produce water for the steam reforming reaction. This oxygen can be produced within the process from water electrolysis.

Preferably, at least some of the hydrogen gas is produced by a water gas shift reaction (preferably a low temperature water-gas-shift reaction), and the water gas shift reaction is carried out on carbon monoxide produced by the steam reforming. Combined with the steam reforming, for every one molecule of carbon derived from the hydrocarbon feedstock, four molecules of hydrogen are generated. Accordingly, this is a very efficient and low cost way of generating hydrogen. The water-gas-shift reaction is typically carried out at a temperature of from 150 to 250 °C (a "low temperature" water-gas-shift). The low temperature water-gas-shift reaction is typically carried out in the presence of a catalyst, typically a transition metal catalyst such as, for example, CuO or CuO-ZnO.

The hydrogen gas and carbon dioxide gas produced by the water-gas-shift reaction may be separated, for example, by the use of Pressure Swing Absorption (PSA). The carbon dioxide may be sent for carbon capture and storage (CCS), or alternatively may be stored for subsequent use in a Sabatier reaction.

Carbon dioxide produced in the low temperature water gas shift reaction is preferably subjected to a Sabatier reaction to produce methane and water. Catalysts for use in the Sabatier reaction include, for example, nickel (see, for example, S Fujita , H Terunuma , M Nakamura , N Takezawa. Mechanisms of methanation of carbon monoxide and carbon dioxide over nickel. Ind. Eng. Chem. Res., 1991, 30 (6), pp 1146-1151, the disclosure of which is hereby incorporated by reference) and ruthenium (see, for example, L Kiewidt, J Thöming, Predicting optimal temperature profiles in single-stage fixed-bed reactors for CO2-methanation, Chemical Engineering Science, Volume 132, 18 August 2015, Pages 59-71, the disclosure of which is hereby incorporated by reference), such as alumina supported ruthenium. While the cost of ruthenium catalysts is much higher, such catalysts may be able to operate at lower temperatures and with higher yields than nickel catalysts. The Sabatier reaction is typically carried out at elevated temperatures (preferably from 300 to 400 °C) and elevated pressures.

At least some of the methane produced by the Sabatier reaction is preferably recycled to be used as the hydrocarbon feedstock in the steam reforming step. At least some of the water produced by the Sabatier reaction is preferably recycled to be used in the steam reforming step.

In a preferred embodiment:

at least some of the hydrogen gas is produced by steam reforming of a hydrocarbon feedstock, the hydrocarbon feedstock being methane,
at least some of the hydrogen gas is produced by a water gas shift reaction, the water gas shift reaction being carried out on carbon monoxide produced by the steam reforming,
carbon dioxide produced in the water gas shift reaction is subjected to a Sabatier reaction to produce methane and water, and
at least some of the methane produced by the Sabatier reaction is recycled to be used as the hydrocarbon feedstock in the steam methane reforming step.

As well as the advantages described above for these features, the preferred embodiment may also maximise continuous throughput.

In a further preferred embodiment:

at least some of the hydrogen gas is produced by steam reforming of a hydrocarbon feedstock,
at least some of the hydrogen gas is produced by a water gas shift reaction, the water gas shift reaction being carried out on carbon monoxide produced by the steam reforming,
carbon dioxide produced in the water gas shift reaction is subjected to a reaction to produce hydrocarbon and water, and
at least some of the hydrocarbon produced by the reaction is recycled to be used as the hydrocarbon feedstock in the steam reforming step.

The hydrocarbon may comprise, for example, methane, ethane, propane and/or butane. The hydrocarbon may be a gas or a liquid or a solid. As well as the advantages described above for these features, the preferred embodiment may also maximise continuous throughput.

In a preferred embodiment:

at least some of the hydrogen gas provided in step (2) is produced by steam reforming of a hydrocarbon feedstock, the hydrocarbon feedstock being methane,
at least some of the hydrogen gas provided in step (2) is produced by a water gas shift reaction, the water gas shift reaction being carried out on carbon monoxide produced by the steam reforming,
carbon dioxide produced in the water gas shift reaction is subjected to a Sabatier reaction to produce methane and water, and
at least some of the methane produced by the Sabatier reaction is recycled to be used as the hydrocarbon feedstock in the steam methane reforming step.

As well as the advantages described above for these features, the preferred embodiment may also maximise continuous throughput.

Hydrogen for use in the Sabatier reaction is preferably produced via the electrolysis of water. Hydrogen may be generated on-demand via the electrolysis of water, which is easier to store than hydrogen. The hydrogen may be generated when the cost of electricity is cheap (e.g. during periods of oversupply). In addition, the hydrogen may be generated using power from renewable sources such as, for example, wind, solar, tidal and hydroelectric dams. Such renewable sources provide varied outputs depending on the prevailing conditions, and thereby are prone to periods of oversupply. However, since the water for electrolysis and the methane produced by the Sabatier reaction are typically easy to store, the generation of hydrogen and subsequent Sabatier reaction may be carried out solely during such periods of oversupply. This may reduce the cost and/or carbon footprint of the process. In other words, while the SNG production may be a continuous process, the hydrogen generation and subsequent Sabatier reaction may be a batch process restricted to periods in which electricity is cheap and/or from a renewable source. This is a particularly significant advantage of the present invention. In this aspect, the methane input may be considered to act not as a fuel, but as an energy carrier.

In a further preferred embodiment:

at least some of the hydrogen gas is produced by steam reforming of a hydrocarbon feedstock, the hydrocarbon feedstock being methane,
at least some of the hydrogen gas is produced by a water gas shift reaction, the water gas shift reaction being carried out on carbon monoxide produced by the steam reforming,
carbon dioxide produced in the water gas shift reaction is subjected to a Sabatier reaction to produce methane and water,
at least some of the methane produced by the Sabatier reaction is recycled to be used as the hydrocarbon feedstock in the steam methane reforming step, and
hydrogen for use in the Sabatier reaction is produced via the electrolysis of water.

As well as the advantages described above for these features, the preferred embodiment may also maximise continuous throughput.

Oxygen produced via the electrolysis of water may be:

reacted with a feedstock to produce synthesis gas for step (1); and/or
used in the steam reforming step.

Typical synthesis gas production methods, such as, for example, gasification and/or gas-plasma methods, often require a source of highly pure oxygen. Oxygen generated by electrolysis is highly pure, thereby avoiding the need to incur the cost of procuring a source of highly pure oxygen. Use of the oxygen in the steam reformation may allow autothermic production of hydrogen.

In a preferred embodiment, step (3) comprises subjecting the methane-enriched gas to a Sabatier reaction to convert at least a portion of any carbon dioxide contained therein into methane. Typically the majority of any carbon dioxide is converted to methane, more typically substantially all of the carbon dioxide is converted to methane. This may result in the substantially complete conversion of carbon from the synthesis gas into methane. Accordingly, in contrast to prior art SNG production process, the efficiency of the process may be increased. Suitable reaction conditions and catalysts for the Sabatier reaction are given above.

The process is preferably operated at a pressure of at least 5 bar, preferably from 10 to 20 bar. The chemical reactions carried out during the process may exhibit an increased rate and/or yield at such pressures. In addition, compression of the final recovered methane-containing gas is made easier, thereby simplifying the process and/or reducing the cost.

Heat generated by exothermic reactions in the process is preferably recovered by use of a heat exchanger, and the recovered heat is used to pre-warm the synthesis gas prior to step (2) and/or pre-warm the hydrogen-enriched synthesis gas prior to step (3). This may reduce the energy cost of the process. Exothermic reactions include, for example, the water-gas-shift reaction, the methanation reaction and the Sabatier reaction.

The hydrogen-enriched synthesis gas preferably has a ratio of carbon monoxide to carbon dioxide of 3 or more, preferably 5 or more, more preferably 10 or more. Carbon monoxide methanation requires a hydrogen to carbon monoxide ratio of 3:1, whereas the Sabatier reaction requires a hydrogen to carbon dioxide ratio of 4:1. Accordingly, increasing the ratio of carbon monoxide to carbon dioxide in the synthesis gas reduces the amount of hydrogen required to take the process to completion, thereby increasing the efficiency of the process. Following methanation and optionally the Sabatier reaction, the conversion to methane may be substantially complete.

The synthesis gas may be produced by, for example, the gasification and/or plasma treatment and/or pyrolysis and/or combustion and/or thermal conversion of a feedstock material. The feedstock material may be a waste material and/or comprise biomass. The synthesis gas production method preferably controls the formation of tars and/or organosulphide species, so that the synthesis gas is of sufficient quality to be subjected to downstream methanation without poisoning of the catalyst. Suitable methods for controlling the formation of tars and organosulphide species are known in the art (see, for example, M Materazzi, P Lettieri, L Mazzei, R Taylor, C Chapman, Tar evolution in a two stage fluid bed-plasma gasification process for waste valorization, Fuel Processing Technology, Volume 128, December 2014, Pages 146-157 and M Materazzi, P Lettieri, L Mazzei, R Taylor, C Chapman, Reforming of tars and organic sulphur compounds in a plasma-assisted process for waste gasification, Fuel Processing Technology, Volume 137, September 2015, Pages 259-268, the disclosures of which are hereby incorporated by reference).

Preferably the synthesis gas is produced according to the process of EP1896774.

Preferably, the synthesis gas is produced in a waste treatment process comprising:

(i) a gasification step comprising treating the waste in a gasification unit in the presence of oxygen and steam to produce an offgas and a non-airborne, solid char material; and
(ii) a plasma treatment step comprising subjecting the offgas and the non-airborne, solid char material to a plasma treatment in a plasma treatment unit in the presence of oxygen and, optionally, steam, wherein the plasma treatment unit is separate from the gasification unit.

Preferably, high purity oxygen, derived from a Cryogenic air separation unit, (ASU) may be used, rather than from a Pressure Swing Adsorption ASU, as it will contain low levels of nitrogen and will therefore produce a synthesis gas with correspondingly reduced levels of nitrogen which will reduce or even avoid the requirement for nitrogen separation at the SNG refining stage. As discussed above, the high purity oxygen may be generated from the electrolysis step.

Preferably the synthesis gas is produced by a method comprising:

(i) thermally treating a feedstock material to produce a synthesis gas; and
(ii) plasma-treating the synthesis gas in a plasma treatment unit in the presence of additional carbon dioxide to produce a refined synthesis gas,

wherein the additional carbon dioxide is added to the feedstock material during the thermal treatment and/or to the synthesis gas before plasma treatment and/or introduced in the plasma treatment unit. The presence of carbon dioxide helps to maintain the seals on the thermal treatment unit and plasma treatment unit, thereby reducing the addition of oxygen or air into the system, which may disrupt the reactions occurring in the treatment units. It also avoids the introduction of inert diluents, such as nitrogen, which may lower the calorific value of the synthesis gas. Carbon dioxide may also act as an oxidant during gasification. The carbon dioxide added to the feedstock may be derived from the off-gas rich in carbon dioxide recovered from the methane-containing gas. In other words, the off-gas rich in carbon dioxide may be re-cycled back into thermal treatment and/or plasma treatment units.

The process may further comprise combusting the substitute natural gas as a fuel, optionally in combination with at least a portion of natural gas.

There is described herein a process for producing a gaseous or liquid hydrocarbon fuel not according to the invention, the process comprising the steps of:

(1) providing a synthesis gas comprising hydrogen and carbon monoxide;
(2) forming a hydrogen-enriched synthesis gas;
(3) subjecting the hydrogen-enriched synthesis gas to an alkanation reaction to convert at least a portion of the gas into alkane thereby forming an alkane-enriched stream; and
(4) recovering from the alkane-enriched stream an alkane-containing stream, wherein step (2) comprises providing a hydrogen gas and combining the hydrogen gas with the synthesis gas.

The alkane-containing stream may be used directly as a gaseous or liquid hydrocarbon fuel. The alkane-containing stream may undergo further processing, for example pressurisation and/or purification, prior to being used as a gaseous or liquid fuel. The alkane may include one or more of C1, C2, C3, C4, C5 and C6. Higher alkanes may also be produced. The gaseous or liquid hydrocarbon fuel may be a substitute natural gas. The alkanation reaction may be a methanation reaction. The preferable and optional features of the first aspect apply equally to this aspect.

There is described herein a process for producing a substitute natural gas not according to the invention, the process comprising the steps of:

(1) providing a synthesis gas comprising hydrogen, carbon monoxide and optionally carbon dioxide;
(2) forming a hydrogen-enriched synthesis gas;
(3) subjecting the hydrogen-enriched synthesis gas to a methanation reaction to convert at least a portion of the gas into methane thereby forming a methane-enriched gas; and
(4) recovering from the methane-enriched gas a methane-containing gas, wherein step (2) is carried out without increasing the carbon dioxide content of the synthesis gas.

The preferable and optional features of the first aspect apply equally to this disclosure.

There is described herein a process for producing a substitute natural gas not according to the invention, the process comprising the steps of:

(1) providing a synthesis gas comprising hydrogen and carbon monoxide;
(2) forming a hydrogen-enriched synthesis gas;
(3) subjecting the hydrogen-enriched synthesis gas to a methanation reaction to convert at least a portion of the gas into methane thereby forming a methane-enriched gas; and
(4) recovering from the methane-enriched gas a methane-containing gas,

wherein step (2) is carried out without reaction of the carbon monoxide in the synthesis gas.

The preferable and optional features of the first aspect apply equally to this disclosure.

There is described herein a process for producing a substitute natural gas not according to the invention, the process comprising the steps of:

(1) providing a synthesis gas comprising hydrogen and carbon monoxide;
(2) forming a hydrogen-enriched synthesis gas;
(3) subjecting the hydrogen-enriched synthesis gas to a methanation reaction to convert at least a portion of the gas into methane thereby forming a methane-enriched gas; and
(4) recovering from the methane-enriched gas a methane-containing gas,

wherein during step (2) a water-gas-shift reaction is not carried out on the synthesis gas.

The preferable and optional features of the first aspect apply equally to this disclosure.

There is described herein a process for producing a substitute natural gas not according to the invention, the process comprising the steps of:

(1) providing a synthesis gas comprising hydrogen and carbon monoxide;
(2) forming a hydrogen-enriched synthesis gas;
(3) subjecting the hydrogen-enriched synthesis gas to a methanation reaction to convert at least a portion of the gas into methane thereby forming a methane-enriched gas; and
(4) recovering from the methane-enriched gas a methane-containing gas, wherein:

step (2) comprises providing a hydrogen gas and combining the hydrogen gas with the synthesis gas,
at least some of the hydrogen gas is produced by steam reforming of a hydrocarbon feedstock, the hydrocarbon feedstock being methane,
at least some of the hydrogen gas is produced by a water gas shift reaction, the water gas shift reaction being carried out on carbon monoxide produced by the steam reforming,
carbon dioxide produced in the water gas shift reaction is subjected to a Sabatier reaction to produce methane and water, and
at least some of the methane produced by the Sabatier reaction is recycled to be used as the hydrocarbon feedstock in the steam methane reforming step.

The preferable and optional features of the first aspect apply equally to this embodiment.

The invention will now be described in relation to the following non-limiting figures, in which:

Figure 1 is flow diagram of a conventional SNG production process.

Figure 2 is a flow diagram of a process not according to the present invention.

Figure 3 is a flow diagram of a process according to the present invention.

Figure 4 is a flow diagram of a process according to the present invention.

Figure 5 is a flow diagram of a process according to the present invention.

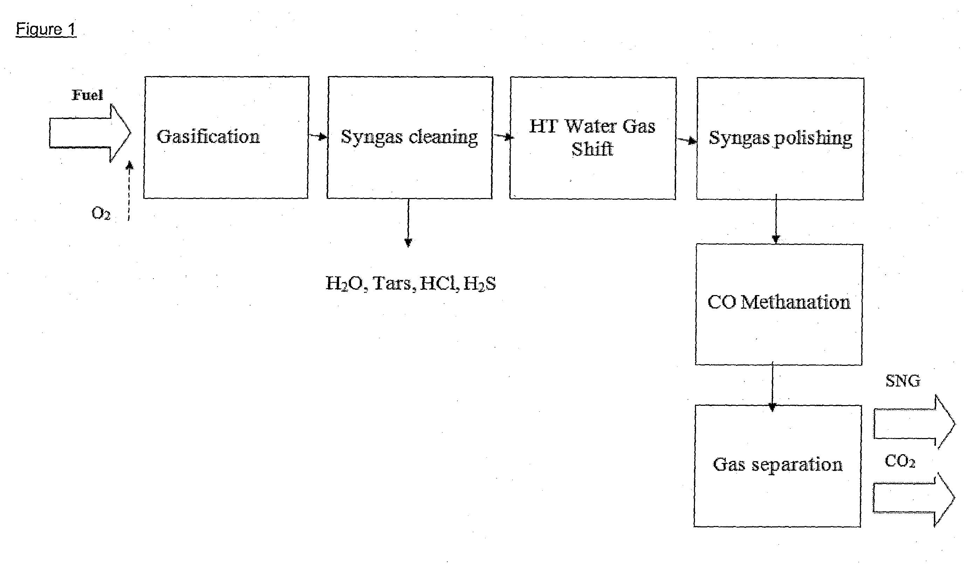

Referring to Figure 1 (a conventional SNG production process), in the gasification step, a carbonaceous solid feed is converted to a synthesis gas using oxygen and steam as the gasification medium. The type of gasifier (e.g. fluid bed, entrained flow, updraft, plasma) and the nature of the feed and feed to oxidant levels employed will impact the quality of the syngas produced. In general, high energy density and friable feedstocks like coal can be pulverised and fed to an entrained flow gasifier which may be operated at high temperatures (i.e. >1200 °C) to produce a syngas with low levels of tars and gaseous hydrocarbons. In contrast, biomass-containing feedstocks are of lower heating values and frequently contain inorganic components in the ash (i.e. soda and potash) which are prone to form low melting point eutectic phases. Waste materials in particular, are heterogeneous in nature, and not amenable to being pulverised. For these types of feedstocks, fluid bed gasifiers are frequently employed due to their ability to handle relatively coarse and chemically heterogeneous materials. These reactors need to be operated at lower temperatures to prevent sintering of the sand causing de-fluidisation of the bed and consequently produce a syngas containing high levels of condensable tars and gaseous hydrocarbon species which can be problematic in the subsequent catalytic water-gas-shift and methanation process stages.

Syngas cleaning is done in a series of stages to remove the various contaminant species that would otherwise poison the downstream catalytic processes. The contaminants that must be removed will vary depending on the chemical composition of the feedstock and the operating conditions within the gasifier but will include sulphur and chloride species, tars and unsaturated hydrocarbons, heavy metals and particulates.

The high temperature water-gas-shift reaction is an exothermic catalytic reaction where CO is reacted with steam to produce hydrogen and $CO_2$.

$$H_2O \text{ (g)} + CO \text{ (g)} \rightarrow H_2 \text{ (g)} + CO_2 \text{ (g)}$$

The purpose is to increase the hydrogen to CO ratio to give the molecular concentrations of hydrogen needed at the methanation stage.

Syngas polishing is carried out to reduce sulphur and chlorine impurities to below 20 ppb, thereby minimising the rate of poisoning of catalysts used in the methanation reaction. The syngas polishing may include, for example, a guard bed (e.g. a ZnO guard bed).

The CO methanation reaction is highly exothermic with the CO reacting with $H_2$ to form $CH_4$ and water according to:

$$3H_2 \text{ (g)} + CO \text{ (g)} \rightarrow CH_4 \text{ (g)} + H_2O \text{ (g)}$$

Additionally, methanation is possible through the reaction of hydrogen and $CO_2$, the Sabatier reaction, especially at high $CO_2$ and low CO concentrations:

$$4H_2 \text{ (g)} + CO_2 \text{ (g)} \rightarrow CH_4 \text{ (g)} + 2H_2O \text{ (g)}$$

There are a number of different reactor design configurations and catalyst materials that may be applied, depending on the specific process chemistry and thermal rating of the facility.

In the gas separation stage, the methane is upgraded using either physical or chemical liquid absorption techniques or Pressure Swing Adsorption (PSA). The liquid absorption technologies may be used for removal of $CO_2$ from the product stream. PSA may additionally be used for separating nitrogen and other impurities from the gas. In many coal SNG applications the $CO_2$ separation is conducted prior to the methanation stage. Additional stages including for example methanation of residual $CO_2$ by the Sabatier reaction may be required to ensure the gas is of sufficient quality for injection into the distribution grid.

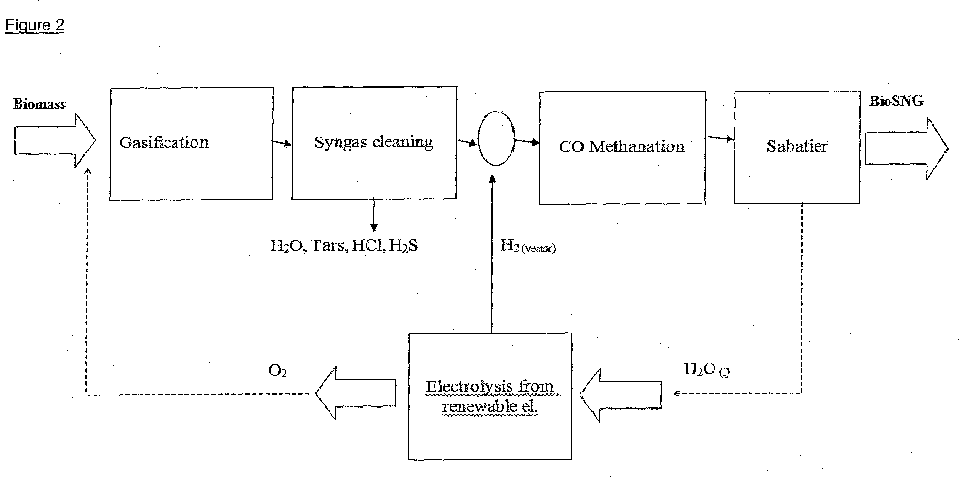

Figure 2 shows a schematic of a process according to an embodiment not according to the present invention. In contrast to the process shown in Figure 1, no water-gas-shift reaction is carried out. The ratio of hydrogen to carbon monoxide is instead increased by externally generating hydrogen gas and then adding the hydrogen gas to the synthesis gas. The hydrogen gas is generated by the electrolysis of water, which has been achieved using electricity from renewable sources. Following CO methanation, a Sabatier reaction is carried out in order to convert any carbon dioxide contained in the synthesis gas into methane and water. Accordingly, substantially the entire carbon content (carbon monoxide and carbon dioxide) of the synthesis gas is converted to methane. Water generated by the Sabatier reaction is used for hydrogen generation via electrolysis. Oxygen generated by the electrolysis is used in the gasification step.

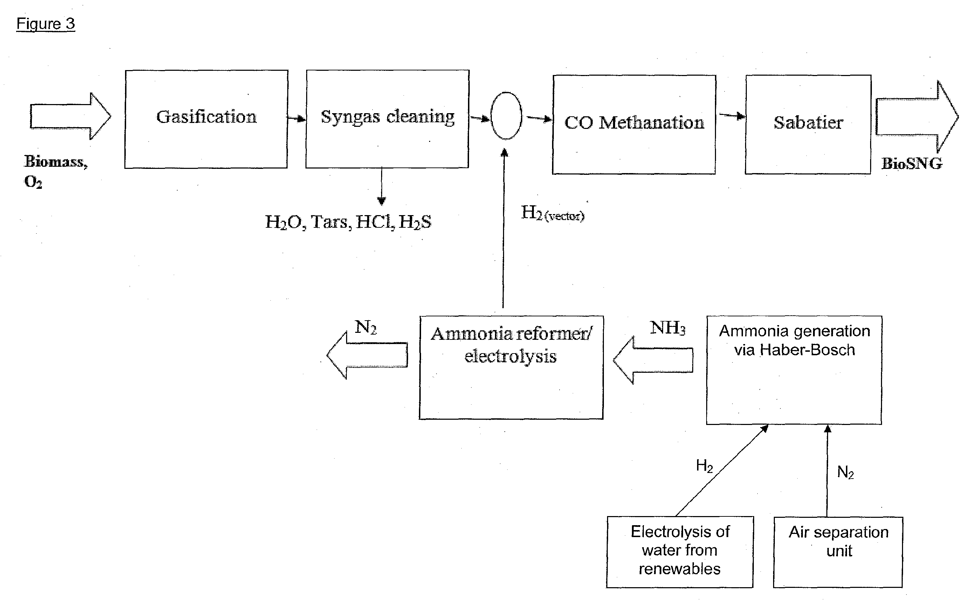

Figure 3 shows a similar process to that shown in Figure 2. However, in this case, hydrogen for use in the CO methanation step is generated by the reformation and/or electrolysis of ammonia according to the present invention. The ammonia is generated on-site using the Haber-Bosch process. The hydrogen for the Haber-Bosch process is generated by the electrolysis of water using renewable sources of electricity. The nitrogen is obtained by use of an air separation unit.

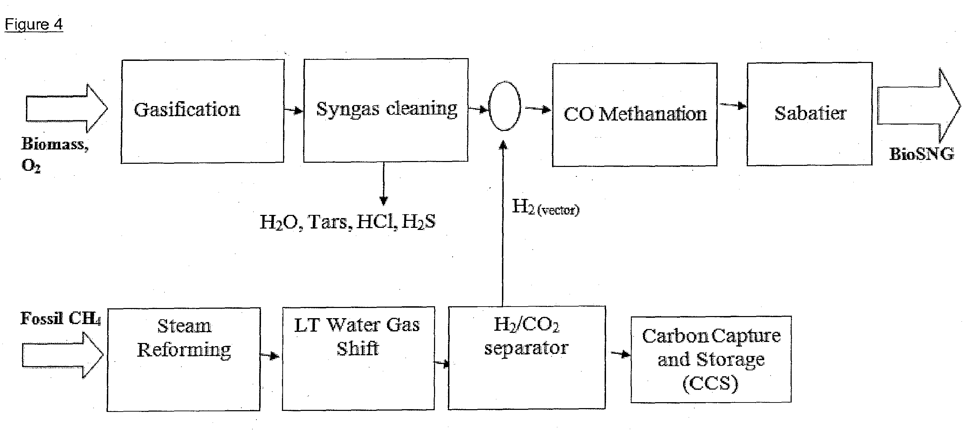

Figure 4 shows a similar process to that shown in Figures 2 and 3. However, in this case the hydrogen for use in the CO methanation step is generated from fossil hydrocarbons, possibly methane. The methane undergoes a steam reforming step according to the present invention to produce hydrogen and carbon monoxide. Carbon monoxide is then subjected to a low temperature water-gas-shift to generate carbon dioxide and more hydrogen. The hydrogen and carbon are separated, for example, by the use of Pressure Swing Absorption (PSA). The hydrogen is sent to the CO methanation step, whereas the carbon dioxide is sent for carbon capture and storage (CCS).

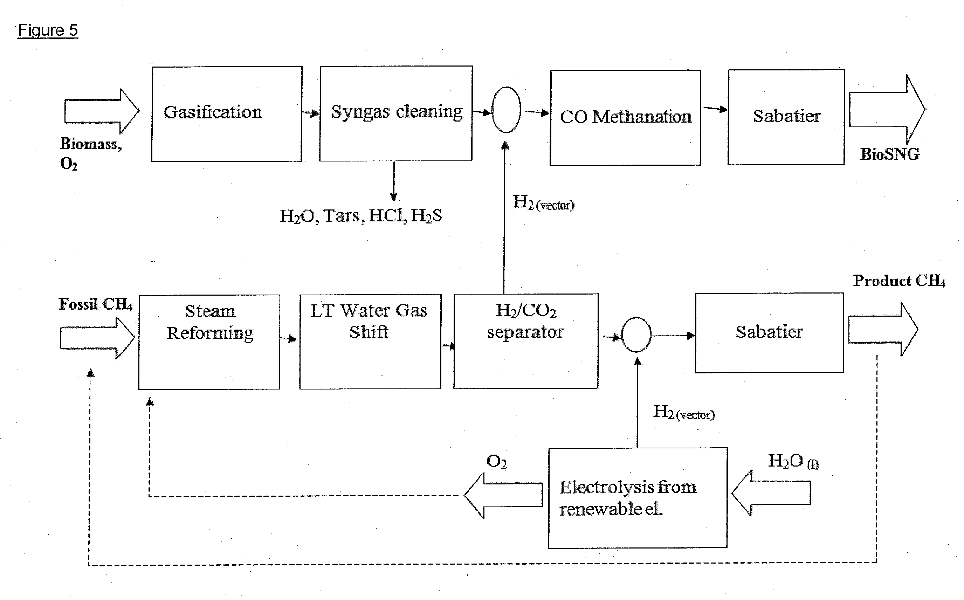

Figure 5 shows a similar process to that shown in Figure 4. In this case, carbon dioxide separated from the hydrogen is subjected to a Sabatier reaction to produce methane. The methane is recycled for use in the steam reforming step. The hydrogen used in this Sabatier reaction is generated from the electrolysis of water.

The foregoing detailed description has been provided by way of explanation and illustration, and is not intended to limit the scope of the appended claims. Many variations in the presently preferred embodiments illustrated herein will be apparent to one of ordinary skill in the art and remain within the scope of the appended claims.

**Claims**

1. A process for producing a substitute natural gas, the process comprising the steps of:

   (1) providing a synthesis gas comprising hydrogen and carbon monoxide;
   (2) forming a hydrogen-enriched synthesis gas;
   (3) subjecting the hydrogen-enriched synthesis gas to a methanation reaction to convert at least a portion of the gas into methane thereby forming a methane-enriched gas; and
   (4) recovering from the methane-enriched gas a methane-containing gas,

wherein step (2) comprises providing a hydrogen gas and combining the hydrogen gas with the synthesis gas, wherein:

(i) at least some of the hydrogen gas is produced by the decomposition of ammonia and/or
(ii) at least some of the hydrogen gas is produced by steam reforming of a hydrocarbon feedstock.

2. The process of claim 1, wherein at least some of the hydrogen gas is produced via the electrolysis of water.

3. The process of claim 1 or claim 2, wherein at least some of the hydrogen gas is produced by the decomposition of ammonia generated using the Haber-Bosch process and/or solid state ammonia synthesis.

4. The process of any preceding claim, wherein at least some of the hydrogen gas is obtained from pyrolysis and/or cracking of hydrocarbons, preferably wherein the pyrolysis and/or cracking of hydrocarbons is part of a carbon black manufacturing method.

5. The process of any preceding claim, wherein at least some of the hydrogen gas is produced by steam reforming of a hydrocarbon feedstock which is methane.

6. The process of any preceding claim, wherein at least some of the hydrogen gas is produced by steam reforming of a hydrocarbon feedstock and at least some of the hydrogen gas is produced by a water gas shift reaction, and wherein the water gas shift reaction is carried out on carbon monoxide produced by the steam reforming.

7. The process of claim 6, wherein carbon dioxide produced in the water gas shift reaction is subjected to a Sabatier reaction to produce methane and water, preferably wherein at least some of the methane produced by the Sabatier reaction is recycled to be used as the hydrocarbon feedstock in the steam methane reforming step.

8. The process of claim 7, wherein hydrogen for use in the Sabatier reaction is produced via the electrolysis of water and, preferably oxygen produced via the electrolysis of water is:

reacted with a feedstock to produce synthesis gas for step (1); and/or
used in the steam reforming step.

9. The process of any preceding claim, wherein step (3) comprises subjecting the methane-enriched gas to a Sabatier reaction to convert at least a portion of any carbon dioxide contained therein into methane.

10. The process of any preceding claim, wherein heat generated by exothermic reactions in the process is recovered by use of a heat exchanger, the recovered heat used to pre-warm the synthesis gas prior to step (2) and/or pre-warm the hydrogen-enriched synthesis gas prior to step (3).

11. The process of any preceding claim, wherein the hydrogen-enriched synthesis gas has a ratio of carbon monoxide to carbon dioxide of 3 or more, preferably 5 or more, more preferably 10 or more.

12. The process according to any of the preceding claims, wherein the synthesis gas is produced by the gasification and/or plasma treatment and/or pyrolysis and/or combustion and/or thermal conversion of a feedstock material, preferably wherein the feedstock is a waste material and/or comprises biomass.

13. The process according to any of the preceding claims, wherein the synthesis gas is produced in a waste treatment process comprising:

(i) a gasification step comprising treating the waste in a gasification unit in the presence of oxygen and steam to produce an offgas and a non-airborne, solid char material; and
(ii) a plasma treatment step comprising subjecting the offgas and the non-airborne, solid char material to a plasma treatment in a plasma treatment unit in the presence of oxygen and, optionally, steam, wherein the plasma treatment unit is separate from the gasification unit.

14. The process according to any preceding claim wherein the synthesis gas is produced by:

(i) thermally treating a feedstock material to produce a synthesis gas; and

(ii) plasma-treating the synthesis gas in a plasma treatment unit in the presence of additional carbon dioxide to produce a refined synthesis gas,

wherein the additional carbon dioxide is added to the feedstock material during the thermal treatment and/or to the synthesis gas before plasma treatment and/or introduced in the plasma treatment unit.

15. The process according to any of the preceding claims, the process further comprising combusting the substitute natural gas as a fuel, optionally in combination with at least a portion of natural gas.

**Patentansprüche**

1. Verfahren zur Herstellung eines Ersatz-Erdgases, wobei das Verfahren die folgenden Schritte umfasst:

(1) Bereitstellen eines Synthesegases, das Wasserstoff und Kohlenmonoxid enthält;
(2) Bilden eines mit Wasserstoff angereicherten Synthesegases;
(3) Unterziehen des mit Wasserstoff angereicherten Synthesegases einer Methanisierungsreaktion, um mindestens einen Teil des Gases in Methan umzuwandeln, wodurch ein mit Methan angereichertes Gas gebildet wird; und
(4) Gewinnen eines methanhaltigen Gases aus dem methanangereicherten Gas,

wobei Schritt (2) das Bereitstellen eines Wasserstoffgases und das Kombinieren des Wasserstoffgases mit dem Synthesegas umfasst, wobei:

(i) zumindest ein Teil des Wasserstoffgases durch die Zersetzung von Ammoniak erzeugt wird und/oder
(ii) zumindest ein Teil des Wasserstoffgases durch Dampfreformierung eines Kohlenwasserstoff-Ausgangsmaterials hergestellt wird.

2. Verfahren nach Anspruch 1, bei dem zumindest ein Teil des Wasserstoffgases durch die Elektrolyse von Wasser erzeugt wird.

3. Verfahren nach Anspruch 1 oder 2, bei dem zumindest ein Teil des Wasserstoffgases durch die Zersetzung von nach dem Haber-Bosch-Verfahren und/oder durch Festphasen-Ammoniaksynthese erzeugtem Ammoniak erzeugt wird.

4. Verfahren nach einem der vorhergehenden Ansprüche, bei dem zumindest ein Teil des Wasserstoffgases durch Pyrolyse und/oder Cracken von Kohlenwasserstoffen gewonnen wird, wobei die Pyrolyse und/oder das Cracken von Kohlenwasserstoffen vorzugsweise Teil eines Verfahrens zur Herstellung von Ruß ist.

5. Verfahren nach einem der vorhergehenden Ansprüche, bei dem zumindest ein Teil des Wasserstoffgases durch Dampfreformierung von Methan als Kohlenwasserstoff-Ausgangsmaterial erzeugt wird.

6. Verfahren nach einem der vorhergehenden Ansprüche, bei dem zumindest ein Teil des Wasserstoffgases durch Dampfreformierung eines Kohlenwasserstoff-Ausgangsmaterials erzeugt wird und zumindest ein Teil des Wasserstoffgases durch eine Wassergas-Shift-Reaktion erzeugt wird, und bei dem die Wassergas-Shift-Reaktion mit durch die Dampfreformierung erzeugtem Kohlenmonoxid durchgeführt wird.

7. Verfahren nach Anspruch 6, bei dem das in der Wassergas-Shift-Reaktion erzeugte Kohlendioxid einer Sabatier-Reaktion unterzogen wird, um Methan und Wasser zu erzeugen, wobei vorzugsweise mindestens ein Teil des durch die Sabatier-Reaktion erzeugten Methans rezykliert wird, um als Kohlenwasserstoff-Ausgangsmaterial in der Dampf-Methan-Reformierungsstufe verwendet zu werden.

8. Verfahren nach Anspruch 7, bei dem Wasserstoff zur Verwendung in der Sabatier-Reaktion durch Elektrolyse von Wasser erzeugt wird und vorzugsweise durch Elektrolyse von Wasser erzeugter Sauerstoff:

mit einem Ausgangsmaterial umgesetzt wird, um Synthesegas für Schritt (1) zu erzeugen; und/oder
im Dampfreformierungsschritt verwendet wird.

9. Verfahren nach einem der vorhergehenden Ansprüche, bei dem in Schritt (3) das mit Methan angereicherte Gas einer Sabatier-Reaktion unterzogen wird, um mindestens einen Teil von darin eventuell enthaltenem Kohlendioxid in Methan umzuwandeln.

10. Verfahren nach einem der vorhergehenden Ansprüche, bei dem die durch exotherme Reaktionen in dem Verfahren erzeugte Wärme unter Verwendung eines Wärmetauschers zurückgewonnen wird, wobei die zurückgewonnene Wärme zum Vorwärmen des Synthesegases vor Schritt (2) und/oder zum Vorwärmen des mit Wasserstoff angereicherten Synthesegases vor Schritt (3) verwendet wird.

11. Verfahren nach einem der vorhergehenden Ansprüche, bei dem das mit Wasserstoff angereicherte Synthesegas ein Verhältnis von Kohlenmonoxid zu Kohlendioxid von 3 oder mehr, vorzugsweise 5 oder mehr, noch bevorzugter 10 oder mehr aufweist.

12. Verfahren nach einem der vorhergehenden Ansprüche, bei dem das Synthesegas durch Vergasung und/oder Plasmabehandlung und/oder Pyrolyse und/oder Verbrennung und/oder thermische Umwandlung eines Ausgangsmaterials erzeugt wird, wobei das Ausgangsmaterial vorzugsweise ein Abfallmaterial ist und/oder Biomasse umfasst.

13. Verfahren nach einem der vorhergehenden Ansprüche, wobei das Synthesegas in einem Abfallbehandlungsverfahren erzeugt wird, das Folgendes umfasst:

(i) einen Vergasungsschritt, der die Behandlung des Abfalls in einer Vergasungseinheit in Gegenwart von Sauerstoff und Dampf umfasst, um ein Abgas und ein nicht schwebendes, festes Verkohlungsmaterial zu erzeugen; und
(ii) einen Plasmabehandlungsschritt, bei dem das Abgas und das nicht schwebende, feste Verkohlungsmaterial einer Plasmabehandlung in einer Plasmabehandlungseinheit in Gegenwart von Sauerstoff und gegebenenfalls Dampf unterzogen werden, wobei die Plasmabehandlungseinheit von der Vergasungseinheit getrennt ist.

14. Verfahren nach einem der vorhergehenden Ansprüche, wobei das Synthesegas erzeugt wird durch:

(i) thermische Behandlung eines Ausgangsmaterials zur Erzeugung eines Synthesegases; und
(ii) Plasmabehandlung des Synthesegases in einer Plasmabehandlungseinheit in Gegenwart von zusätzlichem Kohlendioxid, um ein veredeltes Synthesegas zu erzeugen,

wobei das zusätzliche Kohlendioxid dem Ausgangsmaterial während der Wärmebehandlung und/oder dem Synthesegas vor der Plasmabehandlung zugesetzt und/oder in die Plasmabehandlungseinheit eingeleitet wird.

15. Verfahren nach einem der vorhergehenden Ansprüche, wobei das Verfahren ferner die Verbrennung des Ersatz-Erdgases als Brennstoff, gegebenenfalls in Kombination mit mindestens einem Teil Erdgas, umfasst.

## Revendications

1. Procédé de production d'un gaz naturel de substitution, le procédé comprenant les étapes consistant à :

(1) fournir un gaz de synthèse comprenant de l'hydrogène et du monoxyde de carbone ;
(2) former un gaz de synthèse enrichi en hydrogène ;
(3) soumettre le gaz de synthèse enrichi en hydrogène à une réaction de méthanation pour convertir au moins une partie du gaz en méthane formant ainsi un gaz enrichi en méthane ; et
(4) récupérer à partir du gaz enrichi en méthane un gaz contenant du méthane,

dans lequel l'étape (2) comprend la fourniture d'un hydrogène gazeux et la combinaison de l'hydrogène gazeux avec le gaz de synthèse, où :

(i) au moins une partie de l'hydrogène gazeux est produite par la décomposition de l'ammoniac et/ou
(ii) au moins une partie de l'hydrogène gazeux est produite par vaporeformage d'une charge d'hydrocarbures.

2. Procédé de la revendication 1, dans lequel au moins une partie de l'hydrogène gazeux est produite par électrolyse de l'eau.

**3.** Procédé de la revendication 1 ou 2, dans lequel au moins une partie de l'hydrogène gazeux est produite par la décomposition de l'ammoniac généré en utilisant le procédé Haber-Bosch et/ou la synthèse d'ammoniac à l'état solide.

**4.** Procédé de l'une quelconque des revendications précédentes, dans lequel au moins une partie de l'hydrogène gazeux est obtenue par pyrolyse et/ou craquage d'hydrocarbures, de préférence dans lequel la pyrolyse et/ou le craquage d'hydrocarbures font partie d'un procédé de fabrication de noir de carbone.

**5.** Procédé de l'une quelconque des revendications précédentes, dans lequel au moins une partie de l'hydrogène gazeux est produite par vaporeformage d'une charge d'hydrocarbures qui est le méthane.

**6.** Procédé de l'une quelconque des revendications précédentes, dans lequel au moins une partie de l'hydrogène gazeux est produite par vaporeformage d'une charge d'hydrocarbures et au moins une partie de l'hydrogène gazeux est produite par une réaction de conversion du gaz à l'eau, et dans lequel la réaction de conversion du gaz à l'eau est effectuée sur le monoxyde de carbone produit par vaporeformage.

**7.** Procédé de la revendication 6, dans lequel le dioxyde de carbone produit lors de la réaction de conversion du gaz à l'eau est soumis à une réaction de Sabatier pour produire du méthane et de l'eau, de préférence dans lequel au moins une partie du méthane produit par la réaction de Sabatier est recyclée pour être utilisée comme charge d'hydrocarbures dans l'étape de vaporeformage du méthane.

**8.** Procédé de la revendication 7, dans lequel l'hydrogène destiné à être utilisé dans la réaction de Sabatier est produit par électrolyse de l'eau et, de préférence, l'oxygène produit par électrolyse de l'eau est :

amené à réagir avec une charge pour produire du gaz de synthèse pour l'étape (1) ; et/ou
utilisé dans l'étape de vaporeformage.

**9.** Procédé de l'une quelconque des revendications précédentes, dans lequel l'étape (3) comprend la soumission du gaz enrichi en méthane à une réaction de Sabatier pour convertir au moins une partie de tout dioxyde de carbone qu'il contient en méthane.

**10.** Procédé de l'une quelconque des revendications précédentes, dans lequel la chaleur générée par des réactions exothermiques dans le procédé est récupérée en utilisant un échangeur de chaleur, la chaleur récupérée utilisée pour préchauffer le gaz de synthèse avant l'étape (2) et/ou préchauffer le gaz de synthèse enrichi en hydrogène avant l'étape (3).

**11.** Procédé de l'une quelconque des revendications précédentes, dans lequel le gaz de synthèse enrichi en hydrogène a un rapport de monoxyde de carbone sur le dioxyde de carbone supérieur ou égal à 3, de préférence supérieur ou égal à 5, plus préférablement supérieur ou égal à 10.

**12.** Procédé selon l'une quelconque des revendications précédentes, dans lequel le gaz de synthèse est produit par la gazéification et/ou le traitement au plasma et/ou la pyrolyse et/ou la combustion et/ou la conversion thermique d'une matière de charge, de préférence dans lequel la charge est un déchet et/ou comprend de la biomasse.

**13.** Procédé selon l'une quelconque des revendications précédentes, dans lequel le gaz de synthèse est produit dans un procédé de traitement des déchets comprenant :

(i) une étape de gazéification comprenant le traitement des déchets dans une unité de gazéification en présence d'oxygène et de vapeur pour produire un dégagement gazeux et une matière carbonisée solide non en suspension dans l'air ; et
(ii) une étape de traitement au plasma comprenant la soumission du dégagement gazeux et de la matière carbonisée solide non en suspension dans l'air à un traitement au plasma dans une unité de traitement au plasma en présence d'oxygène et, éventuellement, de vapeur d'eau, où l'unité de traitement au plasma est séparée de l'unité de gazéification.

**14.** Procédé selon l'une quelconque des revendications précédentes, dans lequel le gaz de synthèse est produit par :

(i) traitement thermique d'une matière de charge pour produire un gaz de synthèse ; et

(ii) traitement au plasma du gaz de synthèse dans une unité de traitement au plasma en présence de dioxyde de carbone supplémentaire pour produire un gaz de synthèse raffiné,

dans lequel le dioxyde de carbone supplémentaire est ajouté à la matière de charge pendant le traitement thermique et/ou au gaz de synthèse avant le traitement au plasma et/ou introduit dans l'unité de traitement au plasma.

15. Procédé selon l'une quelconque des revendications précédentes, le procédé comprenant en outre l'étape consistant en la combustion du gaz naturel de substitution en tant que combustible, éventuellement en combinaison avec au moins une partie de gaz naturel.

Figure 1

```
                ┌──────────────┐    ┌──────────────┐    ┌──────────────┐    ┌──────────────┐
Fuel ══════▷    │              │    │              │    │ HT Water Gas │    │              │
                │ Gasification │──▷ │Syngas cleaning│──▷ │    Shift     │──▷ │Syngas polishing│
                │              │    │              │    │              │    │              │
   ⇡            └──────────────┘    └──────────────┘    └──────────────┘    └──────────────┘
   O₂                                      │                                        │
```

$O_2$

$H_2O$, Tars, HCl, $H_2S$

CO Methanation

Gas separation → SNG

CO₂

Figure 1

Figure 2

EP 3 303 524 B1

Figure 3

Gasification → Syngas cleaning → CO Methanation → Sabatier

Biomass, O$_2$

BioSNG

H$_2$O, Tars, HCl, H$_2$S

H$_2$ (vector)

N$_2$

Ammonia reformer/ electrolysis

NH$_3$

Ammonia generation via Haber-Bosch

H$_2$

N$_2$

Electrolysis of water from renewables

Air separation unit

EP 3 303 524 B1

Figure 4

Gasification → Syngas cleaning → ( ) → CO Methanation → Sabatier → BioSNG

Biomass, $O_2$

$H_2O$, Tars, HCl, $H_2S$

$H_2$ (vector)

Fossil $CH_4$ → Steam Reforming → LT Water Gas Shift → $H_2/CO_2$ separator → Carbon Capture and Storage (CCS)

EP 3 303 524 B1

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015120983 A1
- CN 204342750 U
- WO 0021911 A1
- GB 2485923 A
- EP 2843031 A1
- EP 2236457 A1
- US 20100286292 A1
- US 20100272619 A1
- DE 102012200221
- DE 102012015788
- WO 2012067754 A
- DE 102012010542
- WO 2015011503 A
- EP 1896774 A

### Non-patent literature cited in the description

- **GANLEY J ; HOLBROOK J ; MCKINLEY D.** Solid state ammonia synthesis. *Ammonia - Sustainable Emission-Free Fuel Conference,* 15 October 2007
- **S FUJITA ; H TERUNUMA ; M NAKAMURA ; N TAKEZAWA.** Mechanisms of methanation of carbon monoxide and carbon dioxide over nickel. *Ind. Eng. Chem. Res.,* 1991, vol. 30 (6), 1146-1151
- **L KIEWIDT ; J THÖMING.** Predicting optimal temperature profiles in single-stage fixed-bed reactors for CO2-methanation. *Chemical Engineering Science,* 18 August 2015, vol. 132, 59-71
- **M MATERAZZI ; P LETTIERI ; L MAZZEI ; R TAYLOR ; C CHAPMAN.** Tar evolution in a two stage fluid bed-plasma gasification process for waste valorization. *Fuel Processing Technology,* December 2014, vol. 128, 146-157
- **M MATERAZZI ; P LETTIERI ; L MAZZEI ; R TAYLOR ; C CHAPMAN.** Reforming of tars and organic sulphur compounds in a plasma-assisted process for waste gasification. *Fuel Processing Technology,* September 2015, vol. 137, 259-268